(19) Europäisches Patentamt
European Patent Office
Office européen des brevets

(11) **EP 0 711 564 A1**

## (12) EUROPEAN PATENT APPLICATION

(43) Date of publication:
**15.05.1996 Bulletin 1996/20**

(51) Int. Cl.⁶: $A61K\ 39/145$, $C07K\ 14/11$

(21) Application number: **95117311.1**

(22) Date of filing: **07.08.1991**

(84) Designated Contracting States:
**AT BE CH DE DK ES FR GB GR IT LI LU NL SE**

(30) Priority: **08.08.1990 US 564714**

(62) Application number of the earlier application in accordance with Art. 76 EPC: **91915798.2**

(71) Applicant: **UNIVERSITY OF MASSACHUSETTS MEDICAL CENTER**
**Worcester, MA 01655 (US)**

(72) Inventor: **Ennis, Francis A.**
**Shrewsbury, Massachusetts 01545 (US)**

(74) Representative: **Price, Vincent Andrew et al**
**Graham Watt & Co.**
**Riverhead**
**Sevenoaks, Kent TN13 2BN (GB)**

Remarks:
This application was filed on 03 - 11 - 1995 as a divisional application to the application mentioned under INID code 62.

(54) **Cross-reactive influenza A immunization**

(57) This disclosure relates to methods and compositions for stimulating in an individual an influenza A virus protective response which is subtype cross-protective. Influenza A virus NS1 protein, or a T cell epitope thereof, is administered to the individual in an amount sufficient to stimulate the virus protective response.

EP 0 711 564 A1

## Description

Background of the Invention

Influenza A virus is a large RNA-containing animal virus. The protein capsid of the virus is further enclosed in a lipid bilayer-based envelope containing protruding spikes of viral glycoprotein. Three influenza A serotypes have been identified (H1, H2 and H3); the classification based upon differences in the viral glycoprotein

Upon infection by the Influenza A virus, the body produces antibodies to variable regions of the surface glycoproteins hemagglutinin (HA) and neuraminidase (NA). This response results in the production of virus-specific antibodies which constitute the primary defense of the immune system. These antibodies provide immunological pressure which leads to antigenic drift within viral subtypes, as well as shifts between viral subtypes. This relatively high rate of mutagenesis can render vaccine preparations ineffective because the antigenic determinants of the mutated viral proteins can differ significantly from those of the protein used as immunogen resulting in the failure of the body to effectively deal with the infection.

Two central components of the immune system are the B cells and T cells, both of which are lymphocytes. The lymphocyte lineage diverges at the prelymphoblast stage into distinct sublineages. B cells produce and secrete antibody molecules; a process generally referred to as the humoral response. T cells are responsible for a variety of cellular responses referred to generally as cell mediated immune responses.

B cells develop antigen specificity even in the absence of antigen stimulation. It has been estimated, for example, that the preimmune repertoire of a mouse comprises a class having many millions of different antibody molecules. This preimmune repertoire is apparently large enough to insure B cell specificity for almost any potential antigenic determinant.

Current inactivated whole or subunit influenza vaccines provide B cell mediated (humoral) immunity in that they induce antibodies which are directed toward antigenic determinants of the surface glycoproteins of the virus. The first presentation of an influenza antigen to a B cell specific for the antigen (e.g., at the time of vaccination) results in the maturation of the B cell into a plasma cell which is highly specialized for antibody production. Upon a second encounter with the same antigen, a rapid and increased secondary response results. The foreign antigen is bound by the specific antibody followed by clearance of the bound antigen from the bloodstream.

However, in the case of influenza A, the production of virus-neutralizing antibodies provides immunological pressure which leads to antigenic drift within viral subtypes, as well as shifts between viral subtypes. Vaccines which are directed against antigenic sites do not elicit a broadly cross-reactive (i.e. protective against all influenza A virus subtypes) B cell response. Furthermore, the mutations which result from this immunological pressure can render current vaccines ineffective.

T cells comprise a class of cells which, although they do not produce circulating antibodies, do play a central role in the immune system. The T cell class includes helper T cells, cytotoxic T cells and suppressor T cells. Helper T cells function, in part, by augmenting the response of other lymphocytes. For example, helper T cells stimulate activated T lymphocytes, in addition to stimulating B cell activation, by secreting interleukins as well as other soluble factors. Cytotoxic T cells (also referred to as killer T cells), on the other hand, function by destroying cells marked with a particular antigen (e.g. cells infected by virus).

T cells are stimulated by the recognition of a T cell epitope, in combination with a class I or a class II major histocompatiblity (MHC) antigen, on the surface of an antigen presenting cell. Macrophages belong to the class of antigen presenting cells. Macrophages are phagocytes which ingest foreign particles in the body. These cells are capable of ingesting even large micro-organisms such as protozoa. Following ingestion, the antigen presenting cell digests the foreign particle and fragments of the foreign particle are displayed on the surface of the cell.

T cell epitopes differ fundamentally from B cell epitopes. B cell epitopes are antigenic determinants found in the native antigen molecule and not represented in the denatured antigen or fragments thereof. T cell epitopes, on the other hand, are found on unfolded molecules or fragments thereof. Furthermore, the T cell epitopes comprise helper T cell epitopes and cytotoxic T cell epitopes. These epitopes are thought to be contained by distinct, albeit possibly overlapping, portions of the antigen molecule.

Influenza A virus infection continues to cause epidemics of death and tremendous morbidity throughout the world today even though the etiological agent is known. A great deal of effort has been devoted to the development of a vaccine, to little avail. A need exists for an effective influenza A vaccination strategy which could provide cross-subtype immunity from Influenza A viral infection.

Summary of the Invention

This invention relates to Applicants' finding that T cell epitopes of the influenza A NS1 protein are capable of stimulating an influenza A virus protective response, in an individual, which is subtype cross-protective. In a first aspect, the method comprises administering an effective amount of influenza A virus NS1 protein, in combination with a pharmaceutically acceptable carrier, thereby stimulating a T cell response against an NS1 epitope in the individual resulting in an influenza A virus protective response which is subtype cross-protective. A homologue of the NS1 protein in which amino acids have been deleted, inserted or substituted without essentially detracting from the immunological properties thereof, is also effective for this purpose.

In another aspect, the invention relates to a method and composition for immunizing an individual against infection by influenza A virus subtypes by the administration of an effective amount of an influenza A virus T cell epitope in combination with a pharmaceutically acceptable carrier, thereby stimulating a T cell response against the NS1 epitope in the individual resulting in an influenza A protective response which is subtype cross-protective. The T cell epitope can stimulate a cytotoxic T cell response, a helper T cell response, or both. A homologue of the NS1 T cell epitope in which amino acids have been deleted, inserted or substituted without essentially detracting from the immunological properties thereof, is also effective for this purpose.

The invention also relates to an essentially pure oligopeptide having an amino acid sequence corresponding to a T cell epitope of the influenza A NS1 protein. This T cell epitope can stimulate a cytotoxic T cell response, and/or a helper T cell response. Again, a homologue of the NS1 T cell epitope in which amino acids have been deleted, inserted or substituted without essentially detracting from the immunological properties thereof, is also effective for this purpose.

Also disclosed is a method for immunizing an individual against infection by influenza A virus subtypes comprising administering an effective amount of a recombinant virus which expresses the influenza A virus NS1 protein. The individual can also be immunized by administering an effective amount of a recombinant virus which expresses an influenza A virus T cell epitope. These methods are limited to the administration of a recombinant virus which expresses the NS1 protein or a T cell epitope thereof, thereby stimulating a T cell response against a T cell epitope resulting in an influenza A virus protective response which subtype cross-protective.

The methods and compositions described herein provide for a broadly cross-reactive vaccination scheme which is protective against H1, H2 and H3 subtypes of influenza A virus.

Detailed Description of the Invention

As discussed previously, influenza A virus comprises three subtypes. H1, H2 and H3. Applicants' invention relates to methods for immunizing an individual, particularly a human, against infection by any of these subtypes. Although the methods described herein are particularly useful for human immunization, the methods are equally applicable to other mammals. The term "cross-protective" is used in this application to describe immunity against H1, H2 and H3 subtypes.

The gene encoding the influenza A NS1 protein has been isolated, cloned, and expressed in a recombinant vaccinia system (see e.g., Bennink et al., J. Virol. 61:1098-1102 (1987)). Using standard biochemical techniques (e.g. column chromatography) the NS1 protein, having a known molecular weight, can be isolated from cells in which it is expressed. If necessary to attain the desired purity, a hybridoma producing monoclonal antibody specific for NS1 can be generated. Monoclonal antibody produced by this hybridoma can then be used in an affinity capture purification scheme.

Homologues of the NS1 protein in which amino acids have been deleted, inserted or substituted without essentialy detracting from the immunological properties thereof can be generated in a variety of ways. For example, in vitro mutagenic techniques can be used to modify the cloned gene encoding the NS1 protein. Such methods, which are well known to one skilled in the art, can be used to delete, insert or substitute nucleotides in the gene resulting in the deletion, insertion or substitution of amino acids in the encoded product. The immunological properties of the mutagenized encoded product can be assayed using methods such as those described in the Exemplification which follows.

Effective dosages for the stimulation of an influenza A virus protective response are determined empirically with initial dosage ranges based upon historical data for peptide/protein vaccine compositions. As used herein, the term virus protective refers to an immunological response in the individual resulting in the successful control or limitation of infection by influenza A virus subtypes which is clinically observed.

For example, individuals can be administered dosages of NS1 protein ranging from 0.5-500 micrograms. Whether a particular dosage is effective can be determined using well known T cell proliferation and cytotoxicity assays. For example, following administration of the protein to an individual blood is drawn. Cytotoxic T cells are identifiable by $^{51}$Cr release assay (see e.g. Kuwano et al., J. Virol. 140:1264-1268 (1988)). Helper T cells are identifiable by a standard T cell proliferation assay (see e.g. Kurane et al., J. Clin. Invest. 83:506-513 (1989)). The results from these studies are compared with results from the same experiments conducted with T cells from the same individual prior to administration of the antigen. By comparing this data, effective dosage ranges can be determined.

A wide variety of pharmaceutically acceptable carriers are useful. Pharmaceutically acceptable carriers include, for example, water, physiological saline, ethanol polyols (e.g. glycerol or administration is typically parenteral (i.e., intravenous, intramuscular, intraperitoneal or subcutaneous). An adjuvant (e.g., alum) can also be included in the vaccine mixture.

The invention also pertains to a method for immunizing an individual against infection by influenza A virus subtypes by administering a vaccine composition comprising at least one essentially pure T cell epitope of the NS1 protein in combination with a pharmaceutically acceptable carrier. Due to genetic variability between individuals, a single T cell epitope may not stimulate a virus protective response in all individuals to whom it is administered. Therefore, by combining two or more distinct T cell epitopes, the vaccine is more broadly effective. As indicated above, helper T cell epitopes and cytotoxic T cell epitopes are thought to comprise distinct (albeit possibly overlapping) regions of proteins. Cytotoxic T cell epitopes can be distinguished from helper T cell epitopes experimentally using the cytoxicity and proliferation assays described above (helper T cells stimulate proliferation but do not possess cytotoxic activity).

The T cell epitope will be administered as an oligopeptide. Such oligopeptides can be synthesized chemically following identification of the portion of the protein containing the T cell epitope. Alternatively, a truncated portion of the gene encoding the NS1 protein which contains a T cell epitope can be expressed in a cell, and the encoded product can be isolated using known methods (e.g. column chromatography, gel electrophoresis, etc.). In addition, the intact NS1 protein can be treated chemically or enzymatically to generate fragments which contain a T cell epitope. Such fragments can be isolated as described above.

As used herein, the term oligopeptide means any amino acid sequence which is identical or substantially homologous to a portion of the NS1 protein. The expression substantially homologous refers to oligopeptides having an amino acid sequence of an NS1 T cell epitope in which amino acids have been deleted, inserted or substituted without essentially detracting form the immunological properties thereof. This definition includes amino acid sequences of sufficient length to be classified as polypeptides (these terms are not used consistently or with great precision in the literature).

In a preferred embodiment, both a helper T cell epitope and a cytotoxic T cell epitope are administered to the individual. The stimulation of cytotoxic T cells is desirable in that these cells will kill cells infected by influenza A virus. The stimulation of helper T cells is beneficial in that they secrete soluble factors which have a stimulatory effect on other T cells, as well as B cells. As discussed above, due to the genetic variability between individuals, it is preferable to include two or more cytotoxic T cell epitopes and two or more helper T cell epitopes.

Several methods are described in the literature which are useful for the identification of T cell epitopes. For example, DeLisi et al. have suggested that potential epitopic sites may be located by identification of potential amphipathic alpha helical regions in the molecule. DeLisi et al., Proc Natl. Acad. Sci. USA 82:7048 (1987). Bixler et al. describe a strategy of synthesizing overlapping synthetic peptides encompassing an entire protein molecule for delineation of T cell epitopes. Bixler et al., Immunol. Comm. 12:593 (1983); Bixler et al., J. Immunogenet. 11:339 (1984). A synthetic method described by Gysen (Ciba Foundation Symposium 119:130 (1986)) permits synthesis of a large variety of peptides thereby mimicking of a variety of potential binding sites, in turn allowing rapid scanning of a molecule.

More traditional methods, such as enzymatic or chemical digestion of proteins provide peptide fragments which may be readily tested for T cell activity. For example, enzymes such as chymotrypsin, elastase, ficin, papain, pepsin, or trypsin provide limited and predictable fragments by cleavage of specified amino acid linkages: similarly chemical compounds such as N-chlorosuccinimide BPNS-skatole, cyanogen bromide, formic acid, or hydroxylamine, also produce defineable fragments by their action on proteins. The presence of the desired T cell stimulating activity in any given fragment can be readily determined by subjecting purified fragments to a standard T cell proliferation assay, or by anlayzing unpurified fragments with a T cell Western Assay. Young et al., Immunol. 59:167 (1986).

In another embodiment, the gene encoding the NS1 protein, or a portion thereof which contains a T cell epitope, can be cloned into a recombinant virus which expresses the NS1 protein, or T cell epitope containing portion thereof, in the individual to be immunized. An example of such a recombinant virus system is the vaccinia system described by Paoletti et al. (U.S. Patent No. 4,603,112), the disclosure of which is incorporated herein by reference. Other viruses have been described in the literature which have a genome which can accommodate the insertion of foreign DNA such that a protein encoded by the DNA is expressed in vivo. Any such recombinant virus is useful for the practice of this invention.

One skilled in the art will recognize that the compositions described herein can be combined with the components of influenza A vaccines currently in use, thereby resulting in an impoved vaccine. The invention is illustrated further by the following Exemplification.

EXEMPLIFICATION

MATERIALS AND METHODS

Mice

BALB/c mice (H-2$^d$) were purchased from Charles River Breeding Laboratories (Stone Ridge, NY). They were used at 5 to 9 weeks of age.

## Influenza Viruses

Influenza A viruses, A/PR/8 (H1N1), A/BZ (H1N1), A/JAP (H2N2), and A/PC (H3N2), or B/HK, were propagated in 10-day-old embryonated chicken eggs. Infected allantoic fluids were harvested 2 days after infection, aliquoted, and stored at -80° (Kuwano, K. et al., J. Immunol. 140:1264-1268 (1988)).

## Vaccinia Viruses

Vaccinia recombinant viruses containing genes (HA, NP, NS1, and PB2) for A/PR/8 virus were kindly provided by Dr. B. Moss (Bethesda, MD). They were constructed and propagated as previously described (Smith, G.L. et al., Virology 160:336-345 (1987)). Briefly, HeLa cells were infected with virus for 3 days at 37°. Infected cells were pelleted by centrifugation, and resuspended in MEM containing 2% FCS. Three cycles of freezing and thawing were performed and the suspensions were gently sonicated in water for 1 min followed by trypsinization for 30 min at 37°. After centrifugation at 500 rpm for 5 min, supernatants were aliquoted and stored at -80°.

## Cells

The cell lines used in this study, P815 cells (H-$2^d$; mastocytoma) derived from DBA/2 mice, Class 1 MHC molecules, H-$2L^d$- or H-$2D^d$-transfected L929 cell line, and LMI ($K^k$, $L^d$, $D^k$) or DMI ($K^k$, $L^k$, $D^d$), were as described by Weis, J.H. and J.G. Seidman (J. Immunol. 134:1999-2003 (1985)).

## Fusion Protein

D proteins were produced in E. coli as described previously (Yamada, A. et al., Escherichia coli. J. Exp. Med. 162:663-674 (1985); Kuwano, K. et al., J. Immunol. 140:1264-1268 (1988)). Briefly, plasmids containing DNA fragments complementary to the viral RNA of A/PR/8 virus were manipulated to achieve expression of D proteins, which are hybrids of the first 81 amino acids of NS1 fused to the 157 amino acids from the C-terminal end of HA2 through a linker of glutamine-isoleucine-proline. After lysis of the bacteria, two 0.1% deoxycholate extractions and one 1% Triton X-100 extraction were performed to remove contaminating E. coli proteins, and the D protein was solubilized with 4 M urea at 4° for 30 min. The urea was removed by dialysis at 4°. The proteins were stored in 50 mM Tris-HCl, pH 8.0, and 1 mM EDTA. D protein was provided by J.F. Young (Smith, Kline and French Laboratories, Philadelphia, PA).

## CTL Clone

CTL clones were established as described previously (Kuwano, K. et al., J. Immunol. 140:1264-1268 (1988)). Briefly, CTL responder cells were stimulated weekly with A/PR/8 or D protein-pulsed normal syngeneic γ-irradiated spleen cells in the presence of 10% Con A stimulated rat IL2 for several weeks. A limiting dilution was carried out to isolate CTL clones. The B-7 clone was established by stimulation of D protein (Kuwano, K. et al., J. Immunol. 140:1264-1268 (1988)). The A-II clone was stimulated by A/PR/8 virus and grew, at a frequency of growth 2 out of 96 wells, from a well where two responder cells had been seeded. For routine passage of clones, 2 x $10^6$ of clone cell were stimulated weekly by 30 X $10^6$ of A/PR/8 virus or D protein treated γ-irradiated spleen cells in the presence of 10% rat IL2 and 5 x $10^{-5}$ M 2-ME.

## CTL Assay

P815, LMI, or DMI cells (2 X $10^6$) were incubated with 0.5 ml of virus ($10^7$-$10^8$ PFU) in the presence of $^{51}$Cr at 37° for 60 min. After three washings, target cells were incubated for another 1 hr. Then 1 X $10^4$ $^{51}$Cr-labeled target cells were incubated with 1 X $10^4$ effector cells in a total volume of 200 μl in 96-well round bottom microplates for 4 hr at 37°. The supernatant fluids were harvested and specific lysis was determined as percentage specific lysis = 100 X [(release by CTL - spontaneous release)/(maximum release - spontaneous release)].

## Adoptive Transfer of CTL Clone

Cells of the CTL clone (3 X $10^6$) were suspended in 0.5 ml of RPMI 1640 and injected into mice via the tail vein. A preliminary experiment indicated that transfer of 1.0 x $10^6$ cells resulted in significant reductions in mean pulmonary virus titers (0.6 - 0.8 $\log_{10}$PFU) in recipients of clone A-II. Six hours after adoptive transfer of the CTL clone, mice were infected intranasally with $10^3$PFU of virus under ether anesthesia. The lungs of four mice per group were harvested 3 days later for measurement of virus titers.

## Pulmonary Virus Titrations

Virus titrations were performed by plaque formation using MDCK cells as previously described (Kuwano, K. et al., J. Immunol. 140:1264-1268 (1988)). Briefly, infected lungs taken from recipient mice were manually homogenized in 1.5 ml of PBS containing 0.1% BSA. After centrifugation, the lung supernatants were serially 10-fold diluted in PBS. Diluted virus samples (100 µl) were added to confluent MDCK cells in 24-well tissue culture plates and incubated at 37° for 1 hr. Each well then received 1 ml of 1% agar prepared as described earlier (Kuwano, K. et al., J. Immunol. 140:1264-1268 (1988)). After 2 days of incubation, 1 ml of 10% neutral red (GIBCO, Chagrin Falls, OH) in PBS was overlaid on the agar in the wells. Plaques were counted 8 hr later. The results were expressed as the mean $\log_{10}$ PFU/ml of duplicate samples.

## RESULTS

### Cross-Reactivity of Clone A-II Stimulated by A/PR/8 Virus

Four CTL clones were established that were derived from A/PR/8 virus-immune spleen cells of BALB/c mice (H-2$^d$) stimulated by A/PR/8 virus (H1N1). Two of the CTL clones demonstrated H1 subtype-specific lysis of virus-infected target cells. These CTL clones were PB2 protein specific as determined using target cells infected with a vaccinia recombinant virus containing the gene for PB2 of A/PR/8 virus. Clone IE8, representative of two subtype H1-specific clones, is shown as a negative control in Table 2. Clone A-II, which is representative of the other two CTL clones, demonstrated cross-reactive lysis of target cells which were infected with A/PR/8 (H1N1). A/BZ (H1N1), A/JAP (H2N2), or A/PC (H3N2) viruses, but failed to lyse B/HK-infected target cells (Table 1). The B-7 CTL clone (Kuwano, K. et al., J. Immunol. 140:1264-1268 (1988)) was used as a control. B-7 had been stimulated by a fusion protein containing part of the HA2 subunit of A/PR/8 virus and showed subtype H1H2 cross-reactive lysis of target cells that had been infected with A/PR/8 (H1), A/BZ (H1), or A/JAP (H2) viruses. The phenotypes of the cell surface antigens of both the A-II and B-7 clones were Thy1+, Lyt-2+, and L3T4.

TABLE 1

| Virus Specificity of Clone A-II Stimulated by A/PR/8 Virus | | | | | | | |
|---|---|---|---|---|---|---|---|
| Clone | E/T Ratio | % Specific Lysis of P815 Target Cells | | | | | |
| | | A/PR/8 (H1N1) | A/BZ (H1N1) | A/JAP (H2N2) | A/PC (H3N2) | B/HK | Uninfected |
| A-II[a] | 1.0 | 54 | 59 | 58 | 62 | 0 | 0 |
| | 0.5 | 43 | 51 | 43 | 43 | 0 | 0 |
| B-7[b] | 1.0 | 62 | 65 | 43 | 0 | 1 | 0 |
| | 0.5 | 45 | 52 | 32 | 0 | 0 | 0 |

[a]Clone A-II expresses 94% of Thy1.2, 86% of Lyt-2, and 5% of L3T4 surface Ag.
[b]Clone B-7 expresses 97% of Thy1.2, 95% of Lyt-2, and 0% of L3T4 surface Ag.

### CTL Clone A-II is NS1-Protein Specific

To examine the influenza protein recognized by clone A-II, target cells were infected with recombinant vaccinia viruses containing various influenza genes of A/PR/8 virus and were used in CTL assays. As shown in Table 2, clone A-II significantly lysed NS1-VAC-infected and A/PR/8 virus-infected P815 target cells as positive control. However, clone A-II failed to recognize HA-VAC, NP-VAC, PB2-VAC, or parental VAC-infected P815 target cells. Clone B-7 as a negative control lysed HA-VAC-infected target cells as well as A/PR/8 virus-infected target cells, but did not lyse NP-VAC-or VAC-infected target cells. Clone IE8, also derived from A/PR/8 virus-immune spleen cells by repeated stimulation with A/PR/8 virus as described above, recognized PB2-VAC-infected target cells or A/PR/8 virus-infected target cells, but failed to recognize NS1-VAC or HA-VAC-infected target cells; it is also included as a control. These results indicate that CTL clone A-II recognizes the NS1 protein on influenza A virus-infected cells.

Table 2

| Recognition of NS1 Protein of A/PR/8 Virus by Clone A-II | | | | | | | | |
|---|---|---|---|---|---|---|---|---|
| Clone | E/T Ratio | % Specific Lysis of P815 Target Cells | | | | | | |
| | | A/PR/8 | HA-VAC | NP-VAC | NS1-VAC | PB2-VAC | VAC | Uninfected |
| Experiment 1 | | | | | | | | |
| A-II | 1.0 | 55 | -4 | ND | 42 | -4 | ND | -1 |
| | 0.5 | 47 | -4 | ND | 33 | -2 | ND | -2 |
| IE8 | 1.0 | 61 | -3 | ND | 0 | 62 | ND | -1 |
| | 0.5 | 53 | -4 | ND | 0 | 47 | ND | -1 |
| Experiment 2 | | | | | | | | |
| A-II | 3.0 | 78 | 0 | -1 | ND | ND | 0 | 1 |
| | 1.0 | 75 | 1 | -1 | ND | ND | 0 | 1 |
| B-7 | 3.0 | 91 | 91 | -1 | ND | ND | -2 | 0 |
| | 1.0 | 72 | 80 | -1 | ND | ND | -1 | 0 |

Reduction of Pulmonary Virus Titers by Transfer of NS1-Specific CTL Clone

 To examine whether adoptive transfer of NS1 protein-specific CTL clone A-II would reduce virus titers in the lungs of mice infected with influenza viruses, 3 X 10^6 cells of clone A-II were adoptively transferred to BALB/c mice 6 hr prior to influenza infection. Three days later, lungs were removed for titration of influenza viruses. Virus titrations were performed by plaque formation assays in MDCK cells. Similar results were obtained in two experiments with mean decreases in pulmonary virus titers of about 1.0 $\log_{10}$. As shown in Table 3, adoptive transfer of CTL clone A-II significantly reduced the virus titers in the lungs of mice infected with A/PR/8, A/JAP, or A/PC viruses, but did not reduce the virus titer in the lungs of mice infected with B/HK virus. These results reflect the in vitro cross-reactivity of CTL clone A-II shown in Table 1.

Table 3

| Reduction of Pulmonary Virus Titers by Adoptive Transfer of Clone A-II | | | |
|---|---|---|---|
| CTL Clone A-II[a] | RECIPIENTS | | |
| | Virus Challenge | Virus Titer in Lungs[b] | |
| | | Experiment 1 | Experiment 2 |
| + | A/PR/8 (N1N1) | $5.1\pm0.4^c$ | $5.7\pm0.2^f$ |
| - | A/PR/8 | $6.2\pm0.3$ | $6.8\pm0.2$ |
| + | A/JAP(H2N2) | $3.0\pm0.6^d$ | $3.3\pm0.2^g$ |
| - | A/JAP | $4.3\pm0.2$ | $4.4\pm0.2$ |
| + | A/PC(H3N2) | $4.0\pm0.4^e$ | $4.5\pm0.4^h$ |
| - | A/PC | $5.0\pm0.1$ | $5.7\pm0.2$ |
| + | B/HK | $4.1\pm0.1$ | ND |
| - | B/HK | $4.2\pm0.1$ | |

[a]Cells ($3 \times 10^6$) were transferred 6 hr before virus challenge; +, transferred; -, no cells transferred.
[b]Lungs were taken and virus titers were examined by plaque assays in MDCK cells 3 days after virus challenge.
[c]$P<0.01$, Student's t test.
[d]$P<0.02$, Student's t test.
[e]$P<0.005$, Student's t test.
[f]$P<0.005$, Student's t test.
[g]$P<0.0005$, Student's t test.
[h]$P<0.005$, Student's t test.

## MHC Restriction of Target Cell Lysis by Clone A-II

L929 cells (H-$2^k$) transfected with genes encoding H-$2D^d$ (DMI cells) and H-$2L^d$ (LMI cells) were used to examine the MHC restriction of target cells lysis by CTL clone A-II. As shown in Table 4, CTL clone A-II significantly lysed A/PR/8 virus-infected LMI (H-$2L^d$) target cells, but failed to lyse A/PR/8 virus-infected DMI (H-$2D^d$) or A/PR/8 virus-infected DAP(H-$2^k$) target cells

As a control, CTL derived from bulk cultures of A/PR/8 virus-immune BALB/c (H-2d) spleen cells that had been stimulated by A/PR/8 virus in the presence of IL2 for several weeks were also used in this experiment. These virus-stimulated CTL lysed LMI or DMI target cells infected with A/PR/8 virus, but did not kill A/PR/8 virus-infected DAP target cells. It was also observed that the CTL clone A-II was unable to recognize A/PR/8 virus-infected peritoneal exudate cells of C3H.OL mice (H-$2K^d$, $D^k$). These results indicate that recognition by the CTL clone A-II of NS1 on A/PR/8 virus-infected target cells is restricted by the H-$2L^d$ allele.

Table 4

| MHC Restriction of CTL Recognition by CTL Clone A-II | | | | | | | |
|---|---|---|---|---|---|---|---|
| CTL | E/T Ratio | % Specific Lysis of Target Cells | | | | | |
| | | $LMI(H\text{-}2K^k,D^k,L^d)$ | | $DMI(H\text{-}2K^k,D^k,D^d)$ | | $DAP(H\text{-}2K^k,D^k)$ | |
| | | A/PR/8 | None | A/PR/8 | None | A/PR/8 | None |
| A-II | 5.0 | 58 | 1 | 9 | 2 | 0 | 0 |
| | 2.5 | 43 | 1 | 2 | 2 | 2 | 0 |
| A/PR/8 stimulated CTL | 5.0 | 36 | 2 | 24 | 2 | 1 | 1 |
| | 2.5 | 30 | 1 | 11 | 1 | 1 | 1 |

## Claims

1. A method for immunizing an individual against infection by influenza A virus subtypes, the method comprising administering to the individual an effective amount of influenza A virus NS1 protein, or a homologue thereof in which amino acids have been deleted, inserted or substituted without essentially detracting from the immunological properties thereof, in combination with a pharmaceutically acceptable carrier, thereby stimulating a T cell response against NS-1 epitope in the individual, resulting in an influenza A virus protective response which is subtype cross-reactive.

2. A method for immunizing an individual against infection by influenza A virus subtypes, the method comprising Administering an effective amount of a recombinant virus which expresses influenza A virus NS1 protein, or a homologue thereof in which amino acids have been deleted, inserted of substituted without essentially detracting from the immunological properties thereof, thereby stimulating a T cell response against an NS1 epitope in the individual resulting in an influenza A virus protective response which is subtype cross-reactive.

3. A vaccine composition which stimulates an influenza A virus protective response when administered to an individual, the vaccine composition comprising an effective amount of influenza A virus NS1 protein, in combination with a pharmaceutically acceptable carrier.

4. A method for immunizing an individual against infection by influenza A virus subtypes, the method comprising administering an effective amount of an influenza A virus NS1 protein T cell epitope, or a homologue thereof in which amino acids have been deleted, inserted or substituted without essentially detracting from the immunological properties thereof, in combination with a pharmaceutically acceptable carrier, thereby stimulating a T cell response against the NS1 protein T cell epitope in the individual resulting in an influenza A virus protective response which is subtype cross-protective.

5. A method for immunizing an individual against infection by influenza A virus subtypes, the method comprising administering an effective amount of a recombinant virus which expresses an influenza A virus NS1 protein T cell epitope, or a homologue thereof in which amino acids have been deleted, inserted or substituted without essentially detracting from the immunological properties thereof, thereby stimulating a T cell response against the T cell epitope in the individual resulting in an influenza A virus protective response which is subtype cross-protective.

6. A vaccine composition which stimulates an influenza A virus protective response when administered to an individual, the vaccine composition comprising an effective amount of an isolated influenza NS1 protein T cell epitope, or a homologue thereof in which amino acids have been deleted, inserted or substituted without essentially detracting from the immunological properties thereof, in combination with a pharmaceutically acceptable carrier.

7. The method of claim 4 or claim 5 or the vaccine composition of claim 6 which stimulates a cytotoxic T cell response, and/or a helper T cell response.

8. An essentially pure oligopeptide having an amino acid sequence corresponding to a T cell epitope of the influenza A NS1 protein, or a homologue thereof in which amino acids have been deleted, inserted or substituted without essentially detracting from the immunological properties thereof.

9. The essentially pure oligopeptide of Claim 8 which corresponds to a cytotoxic T cell epitope, or which corresponds to a helper T cell epitope.

10. Use of influenza A virus NS1 protein (or a homologue thereof) in which amino acids have been deleted, inserted or substituted without essentially detracting from the immunological properties thereof in the manufacture of a medicament for use in a method according to any one of Claims 1, 2, 4, 5 or 7.

European Patent
Office

**PARTIAL EUROPEAN SEARCH REPORT** Application Number

which under Rule 45 of the European Patent Convention EP 95 11 7311
shall be considered, for the purposes of subsequent
proceedings, as the European search report

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | EP-A-0 366 238 (SMITHKLINE BEECHAM CORP ;ENNIS FRANCIS A (US)) 2 May 1990 <br> * page 12, line 40 - page 17, line 21 * <br> * page 20, line 11 - page 21, line 10 * <br> --- | 1,3,4,6, 7,10 | A61K39/145 <br> C07K14/11 |
| X | WO-A-88 01875 (SMITHKLINE BECKMAN CORP) 24 March 1988 <br> * page 22, line 1 - line 14 * <br> * page 23, line 5 - line 22 * <br> --- | 1,3,4,6, 7,10 | |
| X | EP-A-0 176 493 (SMITHKLINE BECKMAN CORP) 2 April 1986 <br> * page 11, line 15 - page 17, line 10 * <br> --- <br> -/-- | 1,3,4,6, 7,10 | |
| | | | TECHNICAL FIELDS SEARCHED (Int.Cl.5) <br><br> C07K <br> A61K |

## INCOMPLETE SEARCH

The Search Division considers that the present European patent application does not comply with
the provisions of the European Patent Convention to such an extent that it is not possible to carry
out a meaningful search into the state of the art on the basis of some of the claims
Claims searched completely :
Claims searched incompletely :
Claims not searched :
Reason for the limitation of the search:

see sheet C

| Place of search | Date of completion of the search | Examiner |
|---|---|---|
| THE HAGUE | 16 February 1996 | Sitch, W |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C07)

**European Patent** **PARTIAL EUROPEAN SEARCH REPORT** Application Number

Office EP 95 11 7311

| | DOCUMENTS CONSIDERED TO BE RELEVANT | | CLASSIFICATION OF THE APPLICATION (Int.Cl.5) |
|---|---|---|---|
| Category | Citation of document with indication, where appropriate, of relevant passages | Relevant to claim | |
| X | CHEMICAL ABSTRACTS, vol. 113, no. 15, 8 October 1990 Columbus, Ohio, US; abstract no. 130495c, K.KUWANO ET AL 'CROSS-REACTIVE PROTECTION AGAINST INFLUENZA A VIRUS INFECTIONS BY AN NS1-SPECIFIC CTL CLONE' page 499; | 1,3,4,6, 7,10 | |
| Y | * abstract * & VIROLOGY, vol. 178, no. 1, 1990 pages 174-179, --- | 2,5 | |
| Y | S.M.KINGSMAN ET AL 'GENETIC ENGINEERING.CHAPTER 12:'BIOTECHNOLOGY',PAGES 414-456' 1988 , BLACKWELL SCIENTIFIC PUBLICATIONS , OXFORD,GB * page 417 - page 426 * --- | 2,5 | TECHNICAL FIELDS SEARCHED (Int.Cl.5) |
| D,A | THE JOURNAL OF IMMUNOLOGY, vol. 140, no. 4, 15 February 1988 pages 1264-1268, K.KUWANO ET AL 'HA2 SUBUNIT OF INFLUENZA A H1 AND H2 SUBTYPE VIRUSES INDUCES A PROTECTIVE CROSS-REACTIVE CYTOTOXIC T LYMPHOCYTE RESPONSE' ----- | | |

EPO FORM 1503 03.82 (P04C10)

EP 95 11 7311

-C-

Remark: Although claims 1,2,4,5 completely, 7 partially
        are directed to a method of
        treatment of the human/animal
        body (Art. 52(4) EPC) the search
        has been carried out and based on
        the alleged effects of the
        compound/composition